Europäisches Patentamt

**European Patent Office**

Office européen des brevets

(19)

(11) Publication number: **0 042 306**
A2

# (12) EUROPEAN PATENT APPLICATION

(21) Application number: **81302722.4**

(22) Date of filing: **17.06.81**

(51) Int. Cl.³: **C 12 M 1/40,** C 12 M 1/04, C 12 P 17/02 // C12N1/30, C12P7/02, C12P7/04, C12P7/06, C12P7/16, C12P7/22, C12P7/24, C12P7/26

(30) Priority: **17.06.80 US 160273**

(43) Date of publication of application: **23.12.81**
**Bulletin 81/51**

(84) Designated Contracting States: **BE DE FR GB IT NL**

(71) Applicant: **Exxon Research and Engineering Company, P.O.Box 390 200 Park Avenue, Florham Park New Jersey 07932 (US)**

(72) Inventor: **Hou, Ching Tsang, 14 Pendleton Place, Edison, New Jersey (US)**

(74) Representative: **Field, Roger Norton et al, 5 Hanover Square, London W1R OHQ (GB)**

(54) **A low energy continuous process for increasing the oxidative state of an oxidisable organic substrate.**

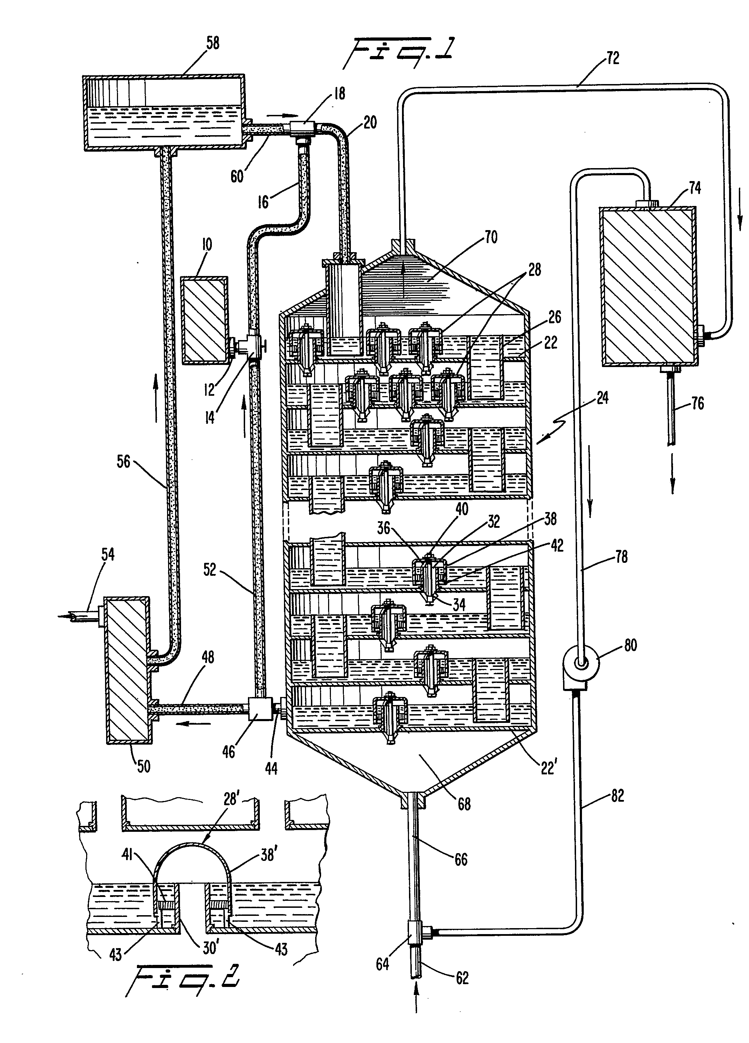

(57) A biocatalytic process for the conversion of an organic substrate comprise establishing a series of separate but intercommunicating sequential contact zones, as shown in Fig. 1, glowing a liquid composition comprising a biocatalyst successively through each of said zones from a liquid inlet zone to a liquid outlet zone, flowing a gas through each of said zones successively through a gas inlet zone to a gas outlet zone, in intimate countercurrent contact with the flowing liquid in each of said zones, flowing an organic substrate successively through each of said zones in intimate, reactive contact with said gas and with the liquid composition containing the biocatalyst, and recovering liquid effluent discharged from the liquid outlet zone and gaseous effluent discharged from the gas outlet zone, said recovered effluent comprising at least some of said organic substrate that has been converted to a different state.

"A low energy continuous process for increasing the oxidative state of an oxidisable organic substrate"

The present invention relates to a continuous practical process for increasing the oxidative state of an oxidizable organic substrate, the process being characterized by low energy requirements. In particular, the process is concerned with the conversion of gaseous hydrocarbons into their respective corresponding alcohols, aldehydes, ketones, epoxides, and acids. More specifically, in one embodiment, the process is concerned with the oxidation of propylene to propylene oxide.

Batch reactions have been described for the conversion of gaseous hydrocarbons into their corresponding alcohols, aldehydes, ketones, epoxides and acids, utilizing biocatalysts in the presence of oxygen. Although suggestions have been made that such processes could be practised on a continuous basis, no such practical process has been described or demonstrated.

The discovery and isolation of certain methylotrophic microorganisms strains, that grow well under aerobic conditions in a culture medium in the presence of methane as the major carbon and energy source, is reported in U.K. patent application GB 2,018,822 A. The methane-grown microbial cells possess a high content of protein. The cells, or enzyme preparations derived from the cells, are said to be useful in converting oxidizable substrates to oxidation products. In particular, $C_1$-$C_6$ alkanes can be converted to alcohols, such as methane to methanol; $C_3$-$C_6$ alkanes can be converted to the corresponding secondary alcohols and methyl ketones; $C_3$-$C_6$ secondary alcohols can be converted to the corresponding methyl ketones; and cyclic hydrocarbons can be converted to cyclic hydrocarbyl alcohols, such as cyclohexane to cyclohexanol; and $C_2$-$C_4$ alkenes selected from ethylene, propylene, butene-1 and butadiene, can be converted to 1,2-epoxides.

Cell-free extracts of certain of these hydrocarbon-utilizing microbes, including bacteria and yeasts, contain a nicotinamide adenine dinucleotide (NAD)-dependent secondary alcohol dehydrogenase (SADH). This enzyme specifically and stoichiometrically oxidizes $C_3$-$C_6$ secondary alcohols, such as 2-propanol and 2-butanol, to their corresponding ketones.

A process for the epoxidation of $C_2$-$C_4$ alpha olefins and dienes, through the action of a particular kind of biocatalyst in the presence of oxygen, is described in U.K. patent application GB 2,019,390 A. The biocatalyst is a particulate fraction of the microorganisms, or an enzyme preparation derived therefrom. The microorganisms are cultivated in a nutrient medium furnishing oxygen and methane or dimethyl ether. The preferred microorganisms are obligative or facultative methylotrophs. The patent publication identifies several particularly preferred strains.

A process is disclosed for the microbiological production of ketones or secondary alcohols from $C_3$-$C_6$ alkanes, and ketones from $C_3$-$C_6$ secondary alcohols, in U.K. patent application GB 2,018,772 A. The process is conducted under aerobic conditions with resting microbial cells derived from a methyloptophic microorganism, or with an enzyme preparation derived from said cells. The microorganism has previously been grown under aerobic conditions in a nutrient medium containing a $C_1$-compound and energy source which is an inducer for the enzyme(s) responsible for producing the ketones. The compound is, for example, methane, methanol, dimethyl ether, methylamine, methyl formate, methyl carbonate, ethanol, propanol or butanol. The term microorganism includes bacteria, protozoa, yeasts and other. Yeast cells, grown as referred to, are illustrated in aerobically converting $C_3$-$C_6$ secondary alcohols. Preparation, isolation and purification of a

novel $C_3$-$C_6$ secondary alcohol dehydrogenase is also disclosed.

The oxidation of alkanes having from 5 to 16 carbon atoms, or of aliphatic alcohols having from 3 to 16 carbon atoms, or of alkenes having from 3 to 8 carbon atoms, or cyclic organic compounds, utilizing a biocatalyst, is described in U.K. patent application GB 2,024,205 A.

In the process described in this application, the biocatalyst may be a culture of a methane-utilizing bacterium of the species Methylosinus trichosporium or an extract thereof containing a methane oxidizing system.

Japanese Kokai 54-017184, published February 8, 1979 to Dalton et al) assigned to National Research Development Corporation, describes the oxidation of straight chain alkanes having more than 3 and less than 9 carbon atoms, of alkenes, and of cyclic organic compounds, utilizing as the biocatalyst a culture of a methane oxidizing bacterium or an extract thereof containing a methane oxidizing system. One way of carrying out the process involves immobilizing the cells on a suitable support material such as glass beads or a gel matrix, to form an immobilized enzyme preparation based on the use of cells as the enzyme source. The immobilized enzyme preparation is maintained in a packed or fluidized bed, and the gaseous substrate is passed through the bed. One of the claimed advantages of this process, when enzyme extracts rather than whole cells were used, is said to be the regeneration in situ of cofactors or other biochemical species required for the enzymatic reaction. The disclosure of this patent application is also incorporated herein by reference.

An early disclosure of the conversion of hydrocarbons of the paraffinic type by bacterial action is described in U.S. patent 2,396,900. The method described in that patent converts normally gaseous paraffinic hydrocarbons into heavy, waxy, oxygenated organic compounds by contacting the hydrocarbons in the presence of oxygen, with an aqueous nutrient solution inoculated with hydrocarbon consuming bacteria of the group consisting of *Bacillus methanicus* and *Bacillus ethanicus*. The patent describes a continuous process carried out in a bubble cap tower. The patent speaks of the bacteria consuming the hydrocarbons. It describes what goes on in the patented process as the synthesis, from light hydrocarbons, of oxygenated organic compounds of the various molecular weights, from low boiling alcohols to waxy acids, esters and alcohols. When the reaction is permitted to proceed to completion, the product is a predominantly heavy waxy body composed of fatty acids and esters thereof. It may be readily saponified.

A later U.S. patent, 3,622,465, describes a process in which the microorganism *Arthrobacter simplex* utilizes $C_3$-$C_{18}$ straight chain hydrocarbons as a principal source of assimilable carbon and energy to produce single cell protein. The fermentation is carried out, in one embodiment of the invention, on a continuous basis in a sieve plate column, using liquified propane gas as the hydrocarbon.

The present invention resides in one preferred

embodiment in a continuous process that increases the oxidative state of an oxidizable organic substrate, comprising establishing a series of separate but interconnected sequential contact zones, flowing a liquid composition comprising a biocatalyst through each of said zones successively from a liquid inlet zone to a liquid outlet zone, flowing an oxidizing gas through each of said zones successively from a gas inlet zone to a gas outlet zone, in intimate, countercurrent contact with the flowing liquid in each of said zones, flowing an organic substrate successively through each of said zones in intimate, reactive contact with said gas and with the liquid composition containing said biocatalyst, and recovering liquid effluent discharged from the liquid outlet zone and gas effluent discharged from the gas outlet zone, the recovered effluents comprising at least some of the oxidizable organic substrate converted to a more advanced state of oxidization.

In this preferred embodiment, the gas-liquid contact apparatus in which the process is carried out is a bubble cap tower. In a very preferred mode of practice of the process, $C_2$-$C_4$ n-alkenes and butadiene, particularly propylene, are converted to the corresponding epoxide.

The process of the invention is particularly useful for converting the gaseous alkanes and alkenes to their corresponding alcohols, aldehydes and acids, i.e., for converting a $C_2$-$C_4$ n-alkene, butadiene, or isobutylene, into the corresponding epoxide, such as, for example, ethylene oxide, propylene oxide, epoxy-butane, epoxy-butene, and epoxy-isobutane. The process can also be conducted to convert methane into methanol, formaldehyde, and formic acid. Gaseous alkanes can also be converted to the corresponding ketones, such as acetone, butanone-2, pentanone-2, and/or primary alcohols, such as ethanol, 1-propanol, 1-butanol, 1-pentanol, secondary alcohols, such as 2-propanol, 2-butanol, 2-pentanol, and the like.

The process of the invention can also be practised for the oxidation of an organic compound ~~selected from the group consisting of~~ which is an alkane having from 5 to 16 carbon atoms inclusive per molecule aliphatic alcohols (primary and secondary) having from 3 to 16 carbon atoms per molecule an alkene having from 3 to 8 carbon atoms per molecule or ~~and~~ a cyclic organic compound such as benzene or a phenol.

In the case of a liquid organic compound substrate, it can be introduced into the upper zone of the reactor, which preferably is a bubble cap tower, along with the liquid composition comprising the biocatalyst.

Also, by controlling process conditions, the process can be utilized for the production of biomass that is useful for the preparation of foodstuffs.

The microorganisms that are useful for producing enzymes for the practice of the invention, whether in whole cell form, or as a crude or purified enzyme, or otherwise, are the methylotrophs or methlotrophic bacteria. These are a recognized genus of microbes.

The following description applies primarily to one embodiment of the invention, wherein the hydrocarbon feedstock is in the gaseous or vapour phase, and the equipment employed is a bubble cap tower.

In the drawing:

Figure 1 is a schematic flow diagram showing one way in which the process of the invention can be practised, making use of a bubble cap tower equipped with downspouts for liquid flow, the bubble cap tower being shown in a fragmentary vertical section, with only a few of the bubble caps being drawn in, and

Figure 2 is a fragmentary section, on an enlarged scale, showing in detail a preferred form of construction for an individual bubble cap for use in the practice of the invention.

Referring now in detail to the drawing a biocatalyst supply tank 10 is connected through a stub line 12 and a tee joint 14 to a supply line 16. The supply line 16 is connected through a fitting 18 to a liquid inlet line 20 that is capable of discharging liquid onto the uppermost tray 22 in a bubble cap tower 24.

The particular bubble cap tower configuration illustrated is one of older design and modest capacity. Each tray, like the uppermost tray 22, is equipped with a downtake pipe 26 of the conventional type, that serves to take overflow from the tray on which it is mounted, and carry it to the tray beneath. Each tray has mounted thereon a plurality of bubble caps 28.

Each bubble cap has a vapor nozzle 30 that is open at both of its ends and that is mounted so that its lower end communicates with the space below the undersurface of the tray. A holddown bolt 32 is disposed to project through a spider 34 that spaces the lower end of the bolt from the lower surface of the tray. The bolt 32 projects upward through the vapor nozzle 30, and a second spider 36 is seated on the upper end of the vapor nozzle 30 about the bolt 32, to space the hood 38 of the bubble cap from the vapor nozzle. The bolt 32 projects through an opening in the hood 38 and a washer-nut assembly 40 is threaded on the upper end of the bolt 32 to hold the bubble cap assembly together and secure it to the tray. Each cap is formed, in conventional fashion, with a plurality of vapor vents 42 about the lower portion of its hood.

In the modified bubble cap structure 28' shown in Fig. 2, the hood 38' is supported on and spaced from the vapor nozzle 30' by a sparger structure 41 that has sufficient structural strength to serve as a spacer but that has openings for the passage of gas or vapors therethrough. The hood 38' is equipped with four legs 43 that space the hood 38' above the tray on which it is mounted, and support it.

As is customary, the downtake pipes 26 in successive trays are staggered from side to side, so that the direction of liquid flow is in one direction on one tray, and in the opposite direction on the next lower tray and so on.

A takeoff pipe 44 is mounted at the lower end of the bubble cap tower 24, positioned to carry off liquid from the lowermost tray. The lowermost tray 22' does not have a downtake pipe, so that the takeoff line 44 provides the only discharge route from the tower for liquid. Some means, designated by the numeral 46 in Fig. 1, is connected to the line 44 to permit the separation of cells or other particulate form of enzyme preparation (when a particulate form is used), from the product-containing liquid effluent. The separation device may be an ultrafilter, a centrifuge, hollow fibers, a conventional filter, or other appropriate device. Means (not shown) are provided to permit recycling of the separated cells or enzyme preparation, after-resuspension, dilution, or the like, as necessary, through a line 52 that is shown in the drawing as communicating with the separation means 46. The separation means 46 is also connected through a line 48 with the product recovery unit 50, so that when the separating means 46 is a filter, for example, the filtrate can be delivered through the line 48 into the product recovery unit 50.

The biocatalyst is recycled in toto, preferably. When its activity drops to less than about 30% to 50% of its original level, it is regenerated before being placed back in service. Regeneration is accomplished, in the case of whole cells, by growth on methane or its metabolites, or other appropriate substrate, as will be described in greater detail presently.

The separation means 46 is connected through a line 52 to the tee 14, so that the line 52 can be used as a recycle line.

The product recovery unit 50 may be, for example, a filter, a hydroclone, a centrifugal separator, or the like, depending upon the characteristics of the product or residue being fed to the product recovery unit. In any case, this unit 50 is provided with a means 54 for removing product from the system, and with a line 56 through which liquid can be pumped to a tank 58, for recycling. The tank 58 is preferably connected to a source of fresh buffer solution, and it should have sufficient capacity to accomodate the recycling liquid. Some agitation means (not shown) is preferably provided to insure homogenity of the material in the tank. The tank 58 is connected through a line 60 to the fitting 18, to permit material from the tank 58 to be fed through the liquid in the line 20 to the upper tray 22 in the bubble cap tower.

In the vapor circulating system, a feed gas line 62 is provided that connects through a tee 64 and a stub line 66 with a generally conical space 68 at the bottom of the tower, beneath the lowermost tray 22'.

Feed gases, such as a mixture of gaseous hydrocarbons and oxygen or air, that are delivered through this system to the tower, then pass up through the tower in known fashion, and eventually collect in the generally conical space 70 at the upper end of the tower. These vapors are led off through a line 72 that connects the upper end of the tower with a product recovery unit 74. This unit is a conventional piece of equipment designed to separate the oxidized product from any residual unreacted gaseous hydrocarbons, and oxygen or air. The product is led off through a line 76, and any residual unreacted substrate gas is recycled through a line 70 to a pump 80, from which it travels through a line 82 to the tee 64, for return to the bottom of the tower.

To practice the process of the invention, a mixture of feed gases, that is, gaseous hydrocarbons, and oxygen or air or other oxidizing gas, is introduced into the bottom of the reactor, which preferably is a bubble cap tower as shown. The gaseous mixture is passed through the reactor, and in the bubble cap tower, this means that it passes through the vapor nozzles of the individual bubble caps, making its way upward through the tower, tray by tray, in countercurrent contact with the liquid that is moving down through the tower. The bubbles are finely divided, and the individual bubble caps are positioned to allow the bubbles from each cap to collide with those from adjacent caps. Intimate contact is thus assured between the gaseous mixture, and the liquid on each tray. At the same time, the liquid is agitated by the bubbles that pass through it. At the top of the tower, residual gas passes off for further processing and for possible recycling.

In the liquid cycle, a biocatalyst and buffer solution are fed into the top of the tower in a continuous stream. The liquid accumulates on the first tray until it overflows the downcomer pipe. It then makes its way down to the next lower tray, and so on, until it reaches the lowermost tray in the tower. It then passes out of the tower for further processing.

The role of the buffer solution is to maintain optimum conditions for the biocatalyst that is employed in the process. The liquid vehicle of the buffer solution also, depending upon the product formed and its characteristics, may collect to product. Alternatively, depending on the nature of the product and the reaction conditions, the product may pass off in the gas leaving the top of the reactor; or part may be in each effluent. The buffer solution also serves to supply energy or cofactors, if necessary, for the biocatalyst. Spent biocatalyst is separated out for recycling or for regeneration and reuse, or is collected for use in feed or for disposal.

In the processes with which the present invention is concerned, the operating temperature overall is generally kept in the range from ambient temperature to about 100°C, but preferably below about 75°C, depending on the vaporizing temperature of the substrate. Heat may be applied to the entire system or to selected parts of it, as desired, for temperature control, or heat exchange may be conducted for either heating or cooling as necessary. In addition, the reactor may be equipped to permit the addition of materials of any kind, as desired, at any tray level. In addition, for very large scale operations, high capacity bubble cap towers may be employed with overflow weirs rather than downtake pipes, and with any desired arrangement for liquid flow on individual trays.

The effective concentration of the biocatalyst depends upon its activity, which tends to decrease over a period of time. Typically, when whole cells are employed, the concentration will be in the range from about 1 mg/cc to about 100 mg/cc of cells in buffer solution, preferably from about 5 mg/cc to about 10 mg/cc. In a cell-free system, the concentration of enzyme may be in the range from about 0.1 mg/cc to about 100 mg/cc of purified or immobilized enzyme preparation in buffer solution.

The product may be recovered from the liquid discharge from the reactor by any convenient means appropriate, such as, for example, distillation, gas purging, and the like, depending upon the particular situation. A part or all of the product can also be recovered from the residual or exhaust gas taken from the top of the reactor. In this case, after stripping off any product present, the residual gas can be recycled by admixture with the incoming feed gases.

The size and capacity of the reactor selected will depend upon the desired scale of production and also on the solubility of the substrate hydrocarbons. A high bubble cap tower ordinarily will have a high partial pressure of the feed gasses and will benefit from higher solubility of the feed gases. The solubilities of the feed gases are usually the rate limiting factors of the reaction.

Substrate hydrocarbon gas or oxygen, oxygen-enriched air, or air can be injected at any desired level in the bubble cap tower to enrich the gas at that level.

For simplicity, Fig. 1 of the drawing shows only a single bubble cap tower. Although it is possible to use only a single tower it is preferred that two or more towers be used in series, with the fresh biocatalyst-buffer solution being directed into the tower from which the feed gas is finally discharging. That is, the fresh supply of feed gas is fed into the bottom of the tower from which the most spent biocatalyst-buffer mixture is being discharged. Thus, recycling of biocatalyst can be most carefully controlled as can be recycling of the effluent substrate gases. Preferably, the total residence time for feed gas in the bubble cap tower should be at least one minute, and need not be more than 30 minutes, depending on the size of the bubble cap tower. The bubble cap tower installation can be designed accordingly.

The pressure differential required to transport the feed gases through a tower reactor system depends on the bubble cap design, depth of liquid per tray, number of trays per tower, and number of towers in use.

The biocatalyst that is employed in the reaction can be in the form of cells, enzyme solutions, or suspensions of immobilized cells or enzymes.

In the following description of preferred embodiments of the practice of the process, methane-induced methylotrophic microorganisms, or enzyme preparations derived therefrom, are used to increase the oxidative state of an oxidizable organic substrate, which is meant to include incorporating oxygen in an organic compound, as in epoxidizing olefins and converting alkanes to alcohols or ketones. In a preferred embodiment of the invention, the process is used to convert propylene to propylene oxide through the use of a biocatalyst.

The term "microorganism" is used herein in its broadest sense to include not only bacteria, but also yeasts, filamentous fungi, actinomycetes and protozoa, and especially the bacteria capable of oxidizing methane.

The term "enzyme preparation" is used to refer to any composition of matter than exhibits the desired oxygenase enzymatic activity. The term is used to refer, for example, to live whole cells, dried cells, cell extracts, and refined and concentrated preparations derived from the cells. Enzyme preparations may be either in dry or liquid form. The term also includes the immobilized form of the enzyme, e.g., the whole cells of the methane grown microorganism, or enzyme extracts, immobilized or bound to an insoluble matrix by covalent chemical linkages, sorption, or entrapment of the enzyme within a gel lattice having pores large enough to allow the molecules of the substrate and of the product to pass freely, but small enough to retain the enzyme.

The term "particulate fraction" refers to the oxygenase enzyme activity in the precipitated or sedimented material when the supernatant after centrifuging broken cells at 10,000 x g. for 30 minutes is centrifuged for 1 hour at 40,000 x g. or greater.

In one preferred embodiment of the invention, practiced for oxidizing propylene to propylene oxide, the preferred biocatalyst is or is derived from, as an enzyme preparation, a methylotrophic organism of the kind referred to in GB 2,019,390 A, and identified as: Methylosinus trichosporium, Methylosinus sporium, Methylocystis parvus, Methylomonas methanica, Methylamonas albus, Methylomonas streptobacterium, Methylomonas agile, Methylomonas rubrum, Methylomonas rosaceus, Methylobacter chroococcum, Methylobacter bovis, Methylobacter capsulatus, Methylobacter vinelandii, Methylococcus capsulatus and Methylococcus capsulatus Strain Texas referred to by D. W. Ribbons, J. Bacteriol, 122, 1351-1363 (1975), and Methylococcus minimus. These methylotropic microorganisms may be used in the form of their whole cells, enzyme extracts thereof or immobilized preparations of those whole cells or enzyme extracts, such as by use of DEAE cellulose, on exchange resins, or porous alumina carriers, for example.

Subcultures of some of such suitable methylotrophic microorganisms have been deposited with the official depository of the United States Department of Agriculture, Agriculture Research Service, Northern Regional Research Laboratory, Peoria, Illinois 61604, by depositing therein subcultures of each, and have received from the depository the individual NRRL strain designations as indicated below. These subcultures have been deposited in accordance with the procedures of the Department of Agriculture without any restriction, and progeny of these strains are available to the public, as follows:

| Culture | USDA Agricultural Research Service Designation | Strain |
|---|---|---|
| Methylosinus trichosphorium | NRRL B-11,196 | A |
| Methylosinus sporium | NRRL B-11,197 | B |
| Methylocystis parvus | NRRL B-11,198 | C |
| Methylomonas methanica | NRRL B-11,199 | D |
| Methylomonas albus | NRRL B-11,200 | E |
| Methylobacter capsulatus | NRRL B-11,201 | F |

Typically, to grow the methylotrophic microorganism and to induce the oxygenase or epoxidation enzyme system, the microorganisms are inoculated into a medium which is contacted with a gas mixture containing methane and oxygen. Methane may be supplied in the form of natural gas. For continuous flow culture the microorganisms may be grown in any suitably adapted fermentation vessel, for example, a stirred baffled fermentor or sparged tower fermentor, which is provided either with internal cooling or an external recycle cooling loop. Alternatively, the process of the present invention may be practised primarily for cell production, as will be described hereafter.

For continuous flow culture in a stirred, baffled fermentor, fresh medium may be continuously pumped into the culture at rates equivalent to 0.02 to 1 culture volume per hour and the culture may be removed at a rate such that the volume of culture remains constant. A gas mixture containing methane and oxygen and possibly carbon dioxide and other gases is contacted with the medium preferably by bubbling continuously through a sparger at the base of the vessel. The source of oxygen for the culture may be air, oxygen or oxygen-enriched air. Spent gas may be removed from the head of the vessel. The spent gas may be recycled either through an external loop or internally by means of a gas inducer impeller. The gas flows and recycle should be arranged to give maximum growth of microorganism and maximum utilization of methane.

The oxyganase enzyme system may be obtained as a crude extract, or as a cell-free particulate fraction, i.e., the material which precipitates or sediments when the supernatant, after centrifuging broken cells at 10,000 x g for 30 mins., is centrifuged for 1 hour at 10,000 x g or greater.

The microbial cells may be harvested from the growth medium by any of the standard techniques commonly used, for example, flocculation, sedimentation, and/or precipitation, followed by centrifugation and/or filtration. The biomass may also be dried, e.g., by freeze or spray drying and may be used in this form for further use in the epoxidation reaction. When using the cell-free enzyme, NADH and a metal (e.g., copper or iron), may be added to enhance the enzyme activity.

The following Examples demonstrate biocatalytic oxidation and biomass production in accordance with the invention. Throughout this application, all parts and percentages are by weight and all temperatures are in degrees Celsius, unless expressly stated to be otherwise.

Oxidation of Hydrocarbons

Example 1

Oxidation of Propylene

A washed cell suspension of the microorganism Methylosinus trichosporium, NRRL B-11,196, hereafter referred to as Strain A, is placed in the biocatalyst supply tank 10. A supply of 0.05M potassium or sodium phosphate buffer solution, pH 7.0, is placed in the tank 58. The valving (not shown) is adjusted so that a mixture of the buffer solution and cell suspension is circulated into the bubble cap tower 24, onto the surface of the uppermost tray 22. Circulation is continued until every tray is filled to capacity, and the liquid from the lowermost tray begins draining out of the tower. The valving is then arranged for complete recycling of the liquid containing the cell suspension. The cell concentration in the cell suspension is maintained at 5-10 mg/cc.

A mixture of gaseous propylene and oxygen-enriched air is pumped into the bottom of the bubble cap tower. The mole ratio of hydrocarbon gas to total oxygen is from about 1:1 to about 1:2. After a short interval of venting of the tower to permit purging the tower of its normal supply of air, the valving is arranged to direct vented gas to flow from the tower through the line 72, into the product recovery unit 74. Initially there is 100% recycling of the gas for a short period of time, to permit the tower to equilibrate.

The tower heat exchange system (not shown) is adjusted to maintain the discharging liquid at about 30°-35°C. After a short time, equilibration quickly is achieved, and the valving is again adjusted so that the liquid from the bottom of the tower is delivered to the product recovery unit 50.

Under these operating conditions, the bulk of the propylene oxide product dissolves in the aqueous vehicle and is carried out of the tower through the line 44 into the product recovery unit 50. It can be simply recovered in that unit by heating the water to drive off the propylene oxide. The water (buffer solution) preferably is then recycled for further use.

Some of the propylene oxide, however, remains in vapor form, and is discharged from the tower at its upper end. It can easily be recovered from the exhaust gases by chilling them to the point where the propylene oxide condenses as a liquid, and thus separates out readily from the other gases.

Other microorganism strains that may be used generate whole cells for use in the cell suspension biocatalyst in the practice of the invention to produce propylene oxide include the following:

TABLE 1

| Strain | Suitable Microorganism Strains |
|---|---|
| B | Methylosinus sporium (NRRL B-11,197) |
| C | Methylosinus parvus (NRRL B-11,198) |
| D | Methylomonas methanica (NRRL B-11,199) |
| E | Methylomonas albus (NRRL B-11,200) |
| F | Methylobacter capsulatus (NRRL B-11,201) |
| G | Methylobacterium organophilim (ATCC 24,886) |
| H | Methylococcus capsulatus (Texas)(ATCC 19,069) |
| I | Methylococcus capsulatus (Bath)(NCIB 11,132) |

In similar fashion, strains A and B can be useful in the form of whole cell biocatalysts for the conversion of ethylene to ethylene oxide; butene-1 to 1-epoxy-butane; and butadiene to 1,2-epoxy-butene. Strain A is believed to effect more rapid conversions, under the stated conditions, with equal weights of cells in the biocatalyst carrier liquid, than Strain B, for the conversion of ethylene to ethylene oxide, propylene to propylene oxide; and butadiene to 1,2-epoxybutene. However, Strain B is believed to effect a more rapid conversion of butene-1 to 1,2-epoxy butane, than Strain A.

Both whole cells and cell-free extracts containing the oxygenase enzyme activity may be used in the epoxidation reaction NADH and metal ion (iron or copper) may be added to enhance activity when the cell-free or purified enzyme preparations are employed.

Cell-free enzyme preparations may be prepared by disintegrating cells in an aqueous suspension, at 4°C, by a single passage through a French pressure cell at 15,000 psi, followed by centrifuging at 5,000 x g for 15 minutes to remove unbroken cells, The supernatant solution is then centrifuged at 40,000 x g for 30 minutes, yielding P (40) and soluble S(40) fractions. The S(40) fraction is subsequently centrifuged at 80,000 x g for 60 minutes, yielding particulate P(80) and soluble S(80) fractions. The two particulate fractions P(40) and P(80) are suspended in 25 mM potassium phosphate buffer, pH 7.0, containing 5 mM magnesium chloride, and then are homogenized at 4°C for use. NADH is necessary for energy in cell-free extracts for optimum oxidation of alkenes. NAD is useful for oxidation of secondary alcohols.

In general, the epoxidation of a $C_2$-$C_4$ n-alkene, lower dienes, or iso-alkenes, selected from the group consisting of ethylene, propylene, butene-1, butadiene, isobutylene, or isoprene, can be carried out where the microorganism, from which the biocatalyst is prepared, belongs to one of the genera <u>Methylosinus</u>, <u>Methylocystis</u>, <u>Methylyomonas</u>, <u>Methylobacter</u>, <u>Methylococcus</u> or <u>Methylobac-terium</u>. The preferred species are selected from <u>Methylosinus trichosporium</u>, <u>Methylosinus sporium</u>, <u>Methylocystis parvus</u>, <u>Methylomonas methanica</u>, <u>Methylomonas albus</u>, <u>Methylomonas streptobactrium</u>, <u>Methylomonas agile</u>, <u>Methylomonas rubrum</u>, <u>Methylomonas rosaceus</u>, <u>Methylobacter chroocuccum</u>, <u>Methylobacter bovis</u>, <u>Methylobacter vinelandii</u>, <u>Methylococcus capsulatus</u>, <u>Methylococcus minimus</u> and <u>Methylobacterium organophilum</u>.

The most preferred strains of microorganisms, for producing the biocatalyst, are those selected from ~~the group consisting of~~ Strains A to I, above.

When the P(40) and P(80) fractions, prepared as described above, of the microorganisms identified below in Table II, are utilized in the process of Example 1, by substituting the particulate cell fraction for the whole cell suspension, a much more concentrated, and thus more highly active, biocatalyst action is available. However, the P(40) fraction contains most of the activity promoting propylene oxidation; the P(80) fraction contains some activity. While most of the enzyme activity appears in the P(40) fraction, on a weight basis, the activity in the P(80) fraction is almost as effective.

TABLE II

CELL-FREE PARTICULATE FRACTIONS OF RESTING MICROBIAL
CELLS EFFECTIVE FOR THE PRODUCTION OF PROPYLENE OXIDE

| Strain | Microorganism |
|---|---|
| | Type 1 Obligate Methylotrophs: |
| J | Methylomonas streptobacterium (NRRL B-11,208) |
| H | Methylococcus capsulatus (Texas, ATCC 19,069) |
| | Type II Obligate Methylotrophs: |
| K | Methylosinus trichosporium (NRRL B-11,202) |
| A | Methylosinus trichosporium (NRRL B-11,196) |
| | Facultative Methylotroph: |
| L | Methylobacterium sp. (NRRL B-11,222) |

Example 2

Oxidation of Methane

When the procedure of Example 1 is generally followed, but with the substitution of either methane or natural gas for propylene as the feed gas, the methane is readily hydroxylated to produce methanol, utilizing whole cell suspensions of microorganisms of the genera, species, and strains identified in Example 1.

In addition to cell suspensions, cell-free particulate fractions of resting microbial cells are effective for the production of methanol from methane. Thus, the P(40) and P(80) fractions can be produced from the microorganisms identified in Table II above, and these are effective in converting methane to methanol. On a weight for weight basis, the P(80) fraction is almost as effective as the P(40) fraction, generally.

General Comments on the Oxidation
of $C_1$-$C_4$ n-alkanes and n-alkenes

Three distinct groups of methane-utilizing organisms are demonstrated to be effective for the oxidation of $C_1$-$C_4$ n-alkanes, and $C_2$-$C_4$ alkenes, utilizing either cell suspensions or cell-free systems.

Thus, when, following the procedure of Example 1, the biocatalyst is made up from the P(40) particulate fraction of the microorganism strain identified in Table II as Strain K, ethylene is converted to ethylene oxide; propylene is converted to propylene oxide; 1-butene is converted to epoxy butane; butadiene is converted to epoxy butene; isobutylene is converted to epoxy isobutane; methane is converted to methanol; and ethane is converted to ethanol.

The optimum pH for the oxidation of methane to methanol is about pH 7.0, and the optimum temperature is about 30°C-40°C.

Since the methane mono-oxyganese from methane-utilizing bacteria appears to be a copper-containing or iron-containing protein, generally the rate of conversion of methane to methanol and of propylene to propylene oxide is increased by the addition of either a copper salt or an iron salt or both. The metal salts are generally added to the buffer solution but may be added to the biocatalyst supply, or to both. Only a very low concentration of metal ion is required, so that buildup should be avoided.

Similarly, the effectiveness of a cell-free fraction of Methylococcus capsulatus (CRL M1, NRRL B-11,219), hereafter Strain M, is found to increase the conversion of methane to methanol and the conversion of propylene to propylene oxide. The cell-free fraction of Strain L is produced by disrupting the cells in a French press following the procedure described earlier.

Generally for the conversion of propylene to prop-lyene oxide by cell suspension, the optimum pH range is from about 6 to about 7, and the optimum temperature is about 30°C-40°C. Although the temperature may be in the range from ambient temperature to about 50°C, it is preferred that the temperature not be above about 40°C, since there is an apparent decrease in oxidation above 40°C, which may be attributable to enzyme instability or to the 35°C boiling point of propylene oxide.

These reactions are inhibited by several metal-binding or metal-chelating agents. Consequently, the presence of such materials should be avoided. Such materials include, for example, 1,10-phenanthroline; alpha, alpha-bipyridyl potassium cyanide, thiosemicarbazide; thiourea, and 8-hydroxy quinoline.

In addition to the microorganisms mentioned above, the biocatalyst for the reactions described in Examples 1 and 2 may be derived from the organisms <u>Methylococcus capsulatus</u> (Bath), Strain I. Biocatalysts derived from this microorganism demonstrate some resistance to inhibition by metal-binding agents.

## Increasing the Oxidative State of Oxidized Substrates as Well as of Hydrocarbons

The $C_3$-$C_6$ methyl ketones, such as, for example, acetone and 2-butanone, can be prepared by the process of the invention, by utilizing as the feed gas a $C_3$-$C_6$ alkane or a $C_3$-$C_6$ secondary alcohol. The biocatalyst can consist of resting microbial cells or an enzyme preparation derived therefrom. The cells are derived from obligate or facultative methylotrophic microorganisms cultivated under aerobic conditions in a mineral-nutrient medium containing an oxygenase and/or dehydrogenase enzyme inducer as the growth and energy source. Example of such inducers include methane, in the case of methane-utilizing methylotrophic microorganisms, and methanol, dimethyl ether, methylamine, methylformate, methylcarbonate, ethanol, propanol, butanol, and the like. For converting the $C_3$-$C_6$ alkanes to the corresponding alcohols, aldehydes, and/or ketones, the microbial cells, or the enzyme preparations derived from the cells, may be derived from obligate or facultative methane-utilizing type methylotrophic microorganisms, but not the methanol-utilizing type. However, to convert the $C_3$-$C_6$ secondary alcohols to the corresponding ketones, the microbial cells or the enzyme preparations derived from the cells may be derived from either the obligate or facultative methane-utilizing or methanol-utilizing type methylotrophic microorganisms; or they may be derived from methylotrophic yeast strains aerobically grown on a plurality of methyl radical-donating compounds such as methanol, methyl amine, methyl formate, methyl carbonate, dimethyl ether, and the like.

Further, for oxidizing the $C_3$-$C_6$ secondary alcohols to their corresponding methyl ketones, the enzyme employed may be an NAD-dependent secondary alcohol dehydrogenase, preferably in the form of a cell-free extract obtained from bacteria or yeast grown on methanol.

The use of whole cells is particularly suitable for the oxidation of the lower molecular weight and/or more lipophilic substrates which may be advantageously absorbed through the cell membrane. In relation to the use of thermophilic methane-oxidizing bacteria such as M. capsulatus, the use of whole cells is particularly advantageous in view of the high growth temperatures e.g., an optimum growth temperature of 45°C for M. capsulatus (Bath), Strain I above, which may be tolerated by the organisms, thus conveniently diminishing cooling requirements.

As an alternative to the use of whole cells, appropriate enzyme extracts of methane-oxidizing bacteria may be used, and may be either membrane associated or soluble extracts. Preferably soluble enzyme extracts, such as those obtained from the Bath strain of M. capsulatus, Strain I, for instance by centrifugation of crude cell extracts, may be employed. Substrates may be oxidized by direct interaction with a solution or suspension of extracts, or more preferably the extracts are first immobilized by attachment to or within a suitable solid phase material, such as glass, cellulose or a synthetic polymeric material, which is contacted with the substrate. The use of extracts is generally applicable to the oxidation of all substrates included within the scope of the invention, though it will be appreciated, however, that when extracts are used the co-factor NADH is usually required for oxidation to proceed.

Processes according to the invention employing enzyme extracts typically compromise as an essential feature the supply, or preferably the regeneration, of co-factors or other biochemical species required or useful in the enzymatic reaction. Any suitable method or means may be employed for regeneration of these biochemical species. For instance, with crude enzyme preparations, formate or formaldehyde, or other suitable electron donor material, together with a catalytic amount of NAD may be used to regenerate NADH for the enzymatic reaction. Catalytic quantities of other required biochemical species may be regenerated similarly.

For a given substrate the rate of oxidation and products obtained may vary having regard to the type of oxidizing agent used e.g., enzyme extract or whole cells, the species of organism and conditions employed during the oxidation process, and the rate of oxidation and products may be optimized as required. Optimum organisms and conditions may be determined by simple experiments which will be apparent to workers skilled in the art, e.g., similar to those described hereinafter in the specific examples. For example, in the oxidation of propene using whole organism of various methane-oxidizing bacteria, organisms of the genus Methylomonas, especially M. albus, have been found to exhibit particularly desirable specific activities for oxidation of the propene to 1,2-epoxypropane.

In one specific embodiment of the present invention, the process of the invention is practised to bring either a $C_3$-$C_6$ alkane or a $C_3$-$C_6$ secondary alcohol into contact, under oxidizing conditions, with resting microbial cells derived from obligate or facultative methylotrophic organisms or enzyme preparations derived from these cells, where the microorganisms have been previously grown under aerobic conditions in a nutrient medium containing methane. The conversion oxidizes the $C_3$-$C_6$ alkanes to alcohols and oxidizes $C_3$-$C_6$ secondary alcohols to $C_3$-$C_6$ methyl ketones. The microorganisms may have been grown under aerobic conditions in a nutrient medium containing methanol, for the conversion of secondary alcohols to the methyl ketones.

The $C_3$-$C_6$ alkanes used in the practice of the invention are preferably linear n-alkanes such as, for example, propane, n-butane, n-pentane, and n-hexane. The $C_3$-$C_6$ secondary alcohols are preferably derived from linear $C_3$-$C_6$ alkanes, most preferably, 2-propanol and 2-butanol.

A preferred group of methane-utilizing methylotrophic microorganisms includes those microorganisms derived from the genera: Methylosinus, Methylocystis, Methylomonas, Methylobacter, Methylococcus and Methylobacterium.

Preferred group of methanol-utilizing methylotrophic microorganisms include those microorganisms derived from the genera: Methanomonas; Pseudomonas; Bacterium; Hyphomicrobium; Achromobacter; Protaminobacter; Vibrio; Rhodopseudomonas; Bacillus; Brevibacterium; Candida; and Hansenula.

Methylotrophic microorganisms species that may be employed include Methylosinus trichosporium, Methylosinus sporium, Methylocystis parvus, Methylomonas methanica, Methylomonas albus, Methylomonas streptobacterium, Methylomonas agile, Methylomonas rubrum, Methylomonas rosaceus, Methylobacter chroococum, Methylobacter bovis, Methylobacter capsulatus, Methylobacter vinelandii, Methylococcus capsulatus (including Methylococcus capsulatus Strain Bath referred to by J. Colby and H. Dalton, J. Biochem., 157 495-497 (1976)), Methylococcus capsulatus Strain Texas referred to by D. W. Ribbons, J. Bacteroil, 122 1351-1363 (1975)), and Methylococcus minimus. These methylotrophic microorganisms may be used in the form of their whole cells, enzyme extracts thereof, or immobilized preparations of those whole cells or enzyme extracts, such as those prepared by the use of DEAE cellulose, an ion exchange resin, or porous alumina, as carriers.

Publicly available subcultures of the species are identified above in Table I. In addition, the following yeasts may be used in the practice of the invention:

TABLE III

| Strain | Strain Name | U.S.D.A. Agriculture Research Center and Designation |
|---|---|---|
| N | Pichia sp. | NRRL Y-11,328 |
| O | Torulopsis sp. | NRRL Y-11,419 |
| P | Klockera sp. | NRRL Y-11,420 |

In addition, the bacterial strains disclosed in Belgian Patent No. 875,512

may be used to practise the invention. Typical bacterial strains that are disclosed in this patent are identified as follows:

TABLE IV

| Strain | Name | U.S.D.A. Agriculture Research Center and Designation |
|---|---|---|
| K | Methylosinus trichosporium | NRRL B-11,202 |
| J | Methylomonas streptobacterium | NRRL B-11,208 |
| Q | Methylomonas agile | NRRL B-11,209 |
| R | Methylococcus capsulatus | NRRL B-11,219 |
| S | Methylobacterium organophilum | NRRL B-11,222 |

Example 3

Conversion of Propane to Acetone

A whole cell suspension of Methylomonas albus (NRRL B-11,200), identified above as Strain E, is used as the biocatalyst and is circulated with a 0.05M phosphate, pH 7.0, buffer solution through a bubble cap tower in the manner described in Example 1. The feed gas mixture is made up of propane and oxygen-enriched air. The temperature of the tower is maintained at about 35°C.

Most of the acetone product, being infinitely soluble in water and having a boiling point of about 56.5°C, leaves the tower in the liquid discharging from the lowermost tray. The acetone product can be separated from the water by a conventional solvent recovery technique, in the product recovery unit. A very small amount of acetone appears in the gaseous discharge from the bubble cap tower, from which it may be recovered by a combination of recycling and washing of the effluent gases.

Similarly, the cell suspensions of the following microorganisms, grown on methane, are useful in the practice of the process of the invention, for converting not only propane to acetone, but also n-butane to 2-butanone, n-pentane to 2-pentanone, and n-hexane to 2-hexanone:

TABLE V

Methylotrophic Microorganism Strain Identification, Whose Resting Cell Suspenions are Useful for the Oxidation of Hydrocarbons to Ketones

| Strain | |
|---|---|
| A | Methylosinus trichosporium (NRRL B-11,196) |
| C | Methylocystis parvus (NRRL B-11,198) |
| D | Methylomonas methanica (NRRL B-11,199) |
| F | Methylobacter capsulatus (NRRL B-11,201) |
| H | Methylococcus capsulatus Texas (ATTC 19,069) |
| T | Methylobacterium organophilum (ATCC 24,886) |

In addition, Strain E can be demonstrated to be useful for the conversion of n-butane to 2-butanone.

Similarly, Strain B can be demonstrated to be useful for the conversion of n-propane to acetone, and for the conversion of n-butane to 2-butanone.

Example 4

Conversion of Secondary Alcohols to Ketones

Following the procedure of the invention, but modified from the procedure of Example 1 in view of the higher boiling point of the substrate, and utilizing a bubble cap tower as a contact device, suspensions of resting cells of the methylotrophic microorganisms of Table V can be demonstrated to be useful for the conversion of 2-propanol to acetone, 2-butanol to 2-butanone, 2-pentanol to 2-pentanone, and 2-hexanol to 2-hexanone. The substrate secondary alcohol in its liquid form is supplied as a continuous stream to the top tray of the tower. Generally the product ketone, which is also of relatively high boiling point, is taken off in liquid form from the lower end of the tower.

To produce the biocatalysts that are useful for this conversion, methane-utilizing methylotrophic microorganisms are each aerobically grown in a methanol-containing nutrient medium instead of methane-containing medium.

After growth of any particular microorganism on a suitable nutrient medium containing methanol as the alcohol dehydrogenase inducer and major source of carbon and energy for growth, the cells are harvested and washed. The cells are harvested by centrifugation at 10,000 x g at 4°C for 30 min. The cell pallet is washed twice with a 0.05 M phosphate buffer at a pH of about 7.0, containing 0.02 M magnesium chloride. The washed cells are then suspended in a phosphate buffer at 0.05 M phosphate buffer at pH 7.0.

The resting cell suspensions of the strains identified in Table V are also operative if grown on methane. Similarly, Strains B and E can be demonstrated to be useful for the oxidation of 2-propanol to acetone and of 2-butanol to 2-butanone. In each case, it is useful to have the resting cell suspensions suspended in a 0.05 M phosphate buffer at a pH of about 7.0.

In an exemplary demonstration of the invention, such a cell suspension (about 10 mg cells/ml) is prepared from cells of Strain E grown on methanol. Isopropanol is added continuously into the recycling liquid composition, to form a 50% isopropanol concentration solution on the uppermost tray. The tower temperature is maintained at about 40°C. Air is used as the feed gas.

The acetone product leaves the bubble cap tower with the liquid effluent. It can be separated from the residual substrate in the liquid effluent and recovered by conventional techniques, such as distillation.

Example 5

Biocatalytic Oxidation of Secondary Alcohols to Ketones

In this example, the biocatalyst once again is a suspension of resting microbial cells. The cells are those of methanol-utilizing methylotrophic microorganisms, grown on methanol.

The procedure of the preceding example is repeated, with the obligate or facultative methanol-using methylotrophic microorganisms each aerobically grown in a methanol-containing nutrient medium. The nutrients in the medium include 0.4% V/V methanol, used as the alcohol dehydrogenase inducer and as the major carbon and energy source for growth. The cells are harvested and washed as in the preceding example. The resting cells are then suspended in a buffer solution as in the preceding example, and are employed in the process of the invention.

## TABLE VI

Identification of Strains Useful for the Conversion of
Secondary Alcohols to Ketones by Cell-Suspensions of
·Methanol-Obligate and Facultative Methanol-Utilizing
Methylotrophs

| Strain | Methylotrophic Microorganism Strain Identification |
|--------|---------------------------------------------------|
| U | Pseudomonas sp. (ATCC 25,262) (Facultative) |
| V | Pseudomonas sp. (ATCC 21,438) (Facultative) |
| W | Pseudomonas sp. (ATCC 21,439) (Obligate) |
| X | Methanomonas methylovora (ATCC 21,852) (Obligate) |

In a modification of the practice of the invention, Strains T and V are each aerobically grown in an alcohol dehydrogenase-inducing growth medium containing methylamine or methylformate as the alcohol dehydrogenase enzyme inducer and growth substrate instead of methane, comprising 0.4 V/V of the growth substrate as the major carbon and energy source. The cells are harvested and washed as previously described, and suspended in a phosphate buffer for use. In both cases, the cell suspensions are effective for the conversion, in each case, of 2-propanol to acetone, of 2-butanol to 2-butanone, of 2-pentanol to 2-pentanone, and of 2-hexanol to 2-hexanone.

## SUMMARY

Considerations Relating to the Conversion of Secondary
Alcohols to Ketones With Cell Suspensions

The optimum pH for the production of ketones does not seem to be critical. The oxidation reaction goes forward at pH values in the range from about 5 to about 10, but generally, a pH of about 8.0 is preferred.

Cell concentration has a direct influence in the rate of conversion. Generally, an increase in conversion rate can be achieved by an increase in concentration of cells.

At least some metal chelating agents appear to exhibit an inhibitory effect on the conversion.

Both cell suspensions and cell-free extracts of $C_1$-compound grown yeasts enzymatically convert $C_3$-$C_6$ secondary alcohols to the corresponding methyl ketones. Further, that cell suspensions of the yeasts: Candida utilis (ATCC 26,387); Hansenula polymorpha (ATCC 26,012); Pichia sp. (NRRL Y-11,328); Torulopsis sp. strain A (NRRL Y-11,419); and Kloeckera sp. strain $A_2$ (NRRL Y-11,420), grown on various $C_1$ compounds (e.g., methanol, methylamine methyl formate), ethanol, and propylamine, can be used to catalyze the oxidation of $C_3$-$C_6$ secondary alcohols to the corresponding methyl ketones.

To derive purified SADH from a yeast, the cells are harvested during exponential growth by centrifugation at 12,000 x g for 15 min. The cell pellet is washed twice with 50 mM phosphate buffer, pH 7. The final pellet is resuspended in the same buffer. Cell suspensions of yeasts grown on ethanol, methylamine, and methylformate can be prepared as described above using 0.4 v/v ethanol, 10 mM methylamine, and 10 mM methylformate, as the sole source of carbon and energy, for example.

The activity of purified yeast-derived SADH is inhibited by sulfhydryl inhibitors and metal-binding agents. The optimum pH of the purified enzyme is about 8. The purified enzyme requires NAD as an electron acceptor.

General:  Conversion to Alcohols

Cell suspensions and cell-free particulate fractions of methane-grown methylotroph microorganisms are capable of catalyzing the conversion of lower alkanes to the corresponding alcohols, both primary and secondary. The conditions for preparing the cell suspensions or the cell-free particulate fractions from methane-grown methylotroph microorganisms are generally the same as described above. The cell-free particulate fraction requires the presence of NADH as an electron donor.

The conversion to the alcohol is inhibited by metal-binding agents. Propylene also inhibits the conversion, which suggests that the propylene and n-alkane (e.g., propane) are competing for the same enzyme system(s). Ascorbate and reduced nicotinamide adenine dinucleotide phosphate (NADPH) can each be utilized as an electron donor in place of NADH for the conversion.

Cell suspensions of the following are useful for the conversion of n-alkanes to secondary alcohols, for example:

TABLE VII

Exemplary Microorganisms Grown on Methane, and
Useful for the Conversion of N-Alkanes to Alcohols

| Strain | Microorganisms |
|--------|----------------|
| A | Methylosinus trichosporium (NRRL B-11) |
| H | Methylococcus capsulatus (Texas, ATCC 19,609) |
| F | Methylobacter capsulatus (NRRL B-11,201) |
| K | Methylosinus sp. (NRRL B-11,202) |
| J | Methylobacterium sp. (NRRL B-11,208) |
| Q | Methylomonas sp. (NRRL B-11,209) |

Example 6

Oxidation of 2-Butanol to 2-Butanone by Means of a Cell-
Free Enzyme Preparation from a $C_1$-Utilizing Microbe

The effectiveness of this biocatalytic demonstration, utilizing cell-free soluble extracts, can be demonstrated with extracts derived from a plurality of different types of microbes. To prepare the cell-free secondary alcohol dehydrogenase (SADH) system, the washed cells are disrupted with a Wave Energy Ultrasonic Oscillator, Model W 201 (Wave Energy Systems, Inc., Newtown, Pa.) and are centrifuged at 20,000 x g for 30 mins. The clear supernatent contains the SADH activity.

All of the cell-free extracts require the addition of a co-factor, NAD, for activity. This appears to be a highly specific cofactor.

Cell-free extracts, useful in the practice of the invention described herein, can be used in the practice of the process of the invention, when the microbes are cultivated on the growth substrates identified respectively in Table VIII below:

TABLE VIII

$C_1$-Utilizing Microorganisms Useful in the Oxidation of 2-Butanol to 2-Butanone by Cell-Free Soluble Extracts

| Strain | Microbes Obligate Methylotrophs | Growth Substrate |
|---|---|---|
| | Type 1 membrane structure | |
| A | Methylosinus trichosporium (NRRL B-11,196) | $CH_4$ $CH_3OH$ $CH_3NH_2$ $HCOOCH_3$ |
| B | Methylosinus sporium (NRRL B-11,197) | $CH_4$ |
| C | Methylocystic parvus (NRRL B-11,198) | $CH_4$ |
| | Type 2 membrane structure | |
| D | Methylomonas methanica (NRL B-11,199) | $CH_4$ |
| E | Methylomonas albus (NRRL B-11,200) | $CH_4$ |
| J | Methylomonas streptobacterium (NRRL B-11,208) | $CH_4$ |
| Q | Methylomonas agile (NRRL B-11,209) | $CH_4$ |
| R | Methylococcus capsulatus (NRRL B-11,219) | $CH_4$ $CH_3OH$ $CH_3NH_2$ $HCOOCH_3$ |
| F | Methylococcus capsulatus (NRRL B-11,201) | $CH_4$ |
| | Facultative methane-utilizers | |
| L | Methylobacterium organophilum (NRRL B-11,222) | $CH_4$ $CH_3OH$ $CH_3NH_2$ $HCOOCH_3$ |
| G | Methylobacterium organophilum (ATCC 27,886) | $CH_4$ |

TABLE VIII (continued)

| Strain | Microbes | Growth Substrate |
|---|---|---|
| | Obligate Methanol-utilizer | |
| W | Pseudomonas sp. (ATCC 21,439) | $CH_3OH$ |
| X | Methylomonas methylovora (ATCC 21,852) | $CH_3OH$ |
| | Facultative methanol-utilizers | |
| V | Pseudomonas sp. (ATCC 21,438) | $CH_3OH$ |
| U | Pseudomonas Ms. (ATCC 25,262) | $CH_3OH$ |
| | Yeasts | |
| | Candida utilis (ATCC 26,387) | $CH_3OH$ |
| | Hansenula polymorpha (ATCC 26,102) | $CH_3OH$ $CH_3NH_2$ $HCOOCH_3$ |
| | Hansenula polymorpha (NRRL Y-2214) | $CH_3OH$ |
| | Hansenula polymorpha (NRRL Y-2267) | $CH_3OH$ |
| | Hansenula amonala (NRRL Y-336) | $CH_3OH$ |
| | Pichia pastoris (NRRL Y-55) | $CH_3OH$ |
| | Pichia pastoris (NRRL Y-7556) | $CH_3OH$ |

Generally, Strain B, cultured on methanol, is observed to induce an extremely rapid reaction for the conversion of 2-butanol to 2-butonone, compared to the soluble crude extracts of the other $C_1$-utilizers.

In carrying out the reaction, the amount of the cofactor NAD that is consumed is substantial. The molar consumption of NAD is very close to the moles of 2-butanol converted in the oxidation reaction to 2-butanone, for example.

Example 7

Biocatalytic Oxidation Using a Purified Enzyme

In the preceding example, the enzyme preparation described is a crude soluble, cell-free extract. In the present example, a purified secondary alcohol dehydrogenase (SADH) is used.

SADH from an obligate methanol utilizer, Pseudomonas sp. (ATCC 21,439), Strain W, is purified as follows. Cells grown on methanol as the carbon source are suspended in 0.05 M sodium phosphate buffer, at pH 7.0, with 0.5 mM dithiothretol. The cells are then disrupted sonically, and a crude extract is separated by centrifugation.

The crude extract is heat treated at 50°C in a water bath for 10 mins. The resulting precipitate is removed by centrifugation. Protamine sulfate (2% solution in a 0.1 M Tris base) (Buffer A), is added to the supernatant solution with agitation.

After standing, the abstract is centrifuged. The supernatant solution is fractionated with solid ammonium sulfate. The material precipitating between 30% and 60% saturation is collected, and dialyzed overnight against Buffer A.

The dialyzed material is applied to a DEAE cellulose column that has been equilibrated with Buffer A. The SADH activity is eluted. This DEAE cellulose eluate is concentrated by ammonium sulfate fractionation. Material precipitating between 30% and 50% ammonium sulfate saturation is collected by centrifugation and dialyzed overnight against Buffer A. This fraction is washed further and filtered through an Amicon unit with an XM 50 membrane. The concentrated fraction inside the Amicon unit is applied to an Affi-Gel Blue Column that has been equilibrated with Buffer A for affinity chromatography. The column is washed overnight with Buffer A and then is eluted with Buffer A containing 5mM NAD. The SADH activity is recovered in the eluate.

The purified SADH may be used directly for converting $C_3$-$C_6$ secondary alcohols to the corresponding methyl ketones, in the process of the present invention. A source of NAD must be added either to the biocatalyst or to the liquid stream that is being fed to the uppermost tray in the bubble cap tower.

The purification procedure described above may be modified by omitting the heat treatment, which results in higher specific activity.

In using the purified SADH, the optimum pH is from about 8 to about 9, and while the optimum temperature is in the range from about 30°C to about 35°C.

The substrate specificity of purified SADH is highest for 2-propanol and 2-butanol. However, it is effective in catalyzing the oxidation of 2-pentanol, 2-hexanol, acetadehyde, cyclohexanol, butane-1,3-diol, and butane-2,3-diol.

Particulate fractions of the following are exemplary of those methylotrophs useful for converting n-alkanes to alcohols:

TABLLE IX

Hydroxylation of N-alkanes to Alcohols by
Particulate P(40) Fraction of Methylotrophs

| Style Strain | Organisms |
|---|---|
| K | Methylosinus sp. (NRRL B-11,202) |
| H | Methylococcus capsulatus (Texas, ATCC 19,069) |
| A ' | Methylosinus trichosporium (NRRL B-11,196) |
| S | Methylobacterium sp. (NRRL B-11,222) |

a) Particulate P(40) fraction is prepared as follows: Cell-suspensions at 4°C are disinte-grated through a French Pressure cell and centri-fuged at 5,000 x g for 30 min. to remove unbroken bacteria. The supernatant solution is then centrifuged at 40,000 x g for 60 min. at 4°C, yielding the particulate P(40) and soluble S(40) fractions.

The process of the invention is effective for oxidizing n-alkanes, although mixtures of oxidation products are produced. Thus, when cell suspensions of methane-grown Strain A and Strain S, for example, are employed, the products are a plurality of oxidation products including primary and secondary alcohols, methyl ketones and aldehydes, as shown below in Table X.

### TABLE X

#### CONVERSION OF N-ALKANES TO OXIDATION PRODUCTS

| Substrate on Which Strain A or Strain S is Grown | Products |
| --- | --- |
| Methane | Methanol |
| Ethane | Ethanol |
| Propane | 1-Propanol |
| | 2-Propanol |
| | Propanol |
| | Acetone |
| Butane | 1-Butanol |
| | 2-Butanol |
| | 2-Butanone |
| | n-butanol |

Each enzyme preparation favors the production of one conversion product, generally, more than others. The rates can readily be determined by experimental practice of the process. Similarly, optimum temperatures and pH values are readily determinable for each particular reaction system.

### Oxidation of Higher Molecular Weight Organic Compounds

The process of the invention can also be practised for the oxidation of an organic compound which is an alkane having from 5 to 16 carbon atoms inclusive per molecule, an aliphatic alcohol having from 3 to 16 carbon atoms per molecule, an alkene having from 3 to 8 carbon atoms per molecule or a cyclic organic compound such as benzene or a phenol.

Any of the microorganisms previously named, containing a methane oxidizing system, or an enzyme preparation derived therefrom, may be used as the biocatalyst. Preferably, the biocatalyst is derived from a culture of methane-utilizing bacteria of the species

<u>Methylylosinus</u> <u>trichosporium</u>, most preferably, <u>Methylosinus</u> <u>trichosporium</u> (NRRL B-11,196) Strain A.

Example 9

<u>Oxidation of Higher Molecular Weight Organic Compounds</u>

The oxidations listed in Table XI below may be performed utilizing a suspension of cells of <u>Methylosinus</u> <u>trichosporium</u> (NRRL B-11,196), Strain A, cultured with methane as a carbon source.

The cells can be harvested during the late logarithmic phase (batch culture) or during the steady state (continuous culture) by centrifuging at 5,000 g for 30 mins. The cells are then washed twice with 20 mM sodium phosphate buffer (e.g. at pH 7.0) and, after resuspending in the same buffer, they may be stored at a lower temperature, i.e., 0°C or less, until required for use.

For use in the process, the cells are suspended in a 20 mM sodium phosphate buffer, pH 7.0, at 5-10 mg cells per ml. The following conversions can be carried out utilizing such cell-suspensions:

TABLE XI

Oxidation of Higher Organic Compounds

| Conversion No. | Substrate | Oxidation Product(s) |
| --- | --- | --- |
| 1 | Benzene | Phenol |
| 2 | Benzyl alcohol | Benzaldehyde and p-hydroxybenzyl alcohol |
| 3 | o-Cresol | 5-methyl,1,3-benzene-diol |
| 4 | Hexane | Hexan-1-ol |
| 5 | Hexadecane | Hexadecane-1-ol |
| 6 | Cyclohexane | Cyclohexanol and 3-hydroxycyclohexanone |
| 7 | Cyclohexanol | 3-hydroxycyclohexanone |
| 8 | Ethylbenzene | Benzoic acid + Benzyl alcohol-Phenylacetic acid + p-hydroxyethyl-benzene |
| 9 | Propylene | Propylene oxide |
| 10 | Octane | Octan-1-ol |
| 11 | Phenol | Catechol + 1,4-dihydroxy benzene |

TABLE XI (Continued)

| Conversion No. | Substrate | Oxidation Product(s) |
|---|---|---|
| 12 | Pyridene | Pyridene-N-oxide |
| 13 | Toluene | Benzoic acid + hydroxy toluene |
| 14 | Styrene | Styrene epoxide |
| 15 | Naphthalene | 1-Naphthol |
| 16 | iso Propyl benzene | 4-methylbenzoic acid |

Following generally the same procedure, propylene can be used as the substrate, as in Conversion No. 9, with methanol present in the circulating liquid medium as an electron donor.

The biocatalyst may be an enzyme preparation of any desired kind, including whole cells or crude or purified enzyme extract, immobilized in an inert inorganic or organic carrier. A preferred immobilized enzyme preparation is obtained by bonding cells to silanated glass beads using gluteraldehyde.

Biomass Production

The methane-grown microbial cells produced for use in the process of the invention possesses a high content of protein and can be used as such, as feedstuffs. Although methane is the preferred source of carbon and energy, the methylotrophic microorganism strains used in the present invention, including yeast strains, may also be grown on other methyl radical-donating, carbon-containing compounds, including methanol, methylamine, methylformate, methylcarbonate, dimethylether, and the like. This technique may be used for biomass production and also for regeneration of partially spent cells.

As is well known, in commercial processes for the production of microorganisms, it is generally necessary to proceed by stages. Ordinarily, propagation is started by inoculating cells from a slant of a culture into a presterilized nutrient medium usually contained in the flask. In the flask, growth of the microorganisms is encouraged as by shaking for aeration, and the maintenance of suitable temperature. This stage is repeated one or more times in flasks or vessels containing the same or larger volumes of nutrient medium. The mass of microorganisms from the last stage, with or without accompanying culture medium, is introduced into a large scale fermentor, in the conventional process, to produce commercial quantities of the microorganisms or enzymes from the microorganisms. In practising the present invention, the mass of microorganisms from the last culture development stage are grown up through the use of the continuous, countercurrent contact process described above, with concomitant oxidation of substrate.

The composition of the liquid phase for biomass production may typically consist of the solution of mineral salts described by Foster and Davis, J. Bacteriol, 91, 1924-1931 (1966), and in Example 1 of GB 2,019,390 A.

The conditions of fermentation in the process of the present invention may be optimized to produce maximum yields of the microbial cells. Among the parameters that may be adjusted to this end are the selection and concentration of nutrients, the pH, osmotic relationships, degree of aeration, temperature, and the like. It is also important to maintain the purity of the fermentation medium.

The fermentation is generally conducted at a temperature in the range from about 5°C to about 50°C, and preferably at a temperature in the range from about 25°C to about 45°C. Generally the pH is controlled to be in the range from about 4 to about 9, and preferably to about 5.5 to 8.5, and most preferably from about 6.0 to about 7.5. The pressure employed during the fermentation must of course be adequate to permit the use of a countercurrent contact device, preferably a bubble cap tower.

When the objective is to produce cells, the oxidized substrate is recovered but is a by-product.

- 40 -

0042306

Conclusion

The invention in one embodiment is in a low-energy-requiring continuous process for carrying out the oxidation of a substrate tha is in either gaseous or liquid form. Higher pressure increases the solubility of gaseous substrates (i.e. gaseous hydrocarbons and oxygen) and stimulates the reaction of growth of microorganisms. The pressure can be adjusted by the adjusting depth of liquid in each tray and by designing the height of the bubble cap tower appropriately. If the pressure is too high, microbial growth may be affected. Therefore, the pressure is generally maintained at 1 to 5 atmospheres, preferably up to 2 atmospheres.

While a bubble cap tower is a preferred type of equipment, the oxidation processes can also be practiced in any other suitable type of equipment, such as, for example, an immobilized enzyme column. Also, the process practised need not be an oxidation process, but may be any process that utilizes a biocatalyst, such as, for example, isomerization, or one of the enzymatic processes described in the article by S. W. May, Enzyme Microb. Technol. 1979, Vol., 1, pp. 15-22, entitled "Enzymatic Epoxidation Reactions."

- ⊥1 -

0042306

CLAIMS:

1. A continuous biocatalytic process for the conversion of an organic substrate, comprising

establishing a series of separate but interconnected sequential contact zones,

flowing a liquid composition comprising a biocatalyst successively through each of said zones from a liquid inlet zone to a liquid outlet zone,

flowing a gas through each of said zones successively, from a gas inlet zone to a gas outlet zone, in intimate, countercurrent contact with the flowing liquid in each of said zones,

flowing an organic substrate successively through each of said zones in intimate, reactive contact with said gas and with the liquid composition containing said biocatalyst, and

recovering liquid effluent discharged from the liquid outlet zone and gaseous effluent discharged from the gas outlet zone,

said recovered effluents comprising at least some of said organic substrate that has been converted to a different state.

2. A process according to claim 1 wherein the gas comprises a source of oxygen and the organic substrate is converted by the process to a more advanced state of oxidation.

3. A process according to claim 2 wherein the organic substrate is an oxidizable organic substrate and is in gaseous or vapor form, and is applied to the gas inlet zone for flow through said sequential contact zones with said oxidizing gas.

0042306

4. A process according to claim 3 wherein the substrate comprises ethylene that is converted to ethylene oxide.

5. A process according to claim 3 wherein the substrate comprises propylene that is converted to propylene oxide.

6. A process according to claim 2 wherein th organic substrate is an oxidizable organic substrate and is in liquid form, and is supplied to said liquid inlet zone for flow through said sequential contact zones with said biocatalyst liquid composition.

7. A process according to any one of the preceding claims wherein the biocatalyst comprises cells.

8. A process according to claim 7 wherein the biocatalyst comprises purified, immobilized enzyme.

Fig.1

Fig.2

0042306